# EUROPÄISCHE PATENTSCHRIFT

(11) **EP 1 562 984 B1**
(45) Veröffentlichungstag und Bekanntmachung des Hinweises auf die Patenterteilung: **09.08.2006**
(21) Anmeldenummer: 03785634.1
(22) Anmeldetag: 16.10.2003
(51) Int. Cl.: C07K 14/58, G01N 33/68, G01N 33/74, G01N 33/76

(54) **SANDWICH-IMMUNOASSAY ZUR BESTIMMUNG VON PROANP-TEILPEPTIDEN**
SANDWICH IMMUNOASSAY FOR IDENTIFYING PARTIAL PROANP PEPTIDES
DOSAGE IMMUNOLOGIQUE SANDWICH POUR DETECTER DES PEPTIDES PARTIELS PROANP

(30) Priorität: 20.11.2002 DE 10254149
(43) Veröffentlichungstag der Anmeldung: 17.08.2005
(73) Patentinhaber: B.R.A.H.M.S Aktiengesellschaft, 16761 Hennigsdorf (DE)
(72) Erfinder: BERGMANN, Andreas, 12351 Berlin (DE); STRUCK, Joachim, 12161 Berlin (DE)
(74) Vertreter: Andrae, Steffen
(86) Internationale Anmeldenummer: PCT/EP2003/011597
(87) Internationale Veröffentlichungsnummer: WO 2004/046181

(56) Entgegenhaltungen:
- WO-A-00/19207
- MACAULAY HUNTER E F ET AL: "ANALYSIS OF PEPTIDES DERIVED FROM PRO ATRIAL NATRIURETIC PEPTIDE THAT CIRCULATE IN MAN AN INCREASE IN HEART DISEASE" SCANDINAVIAN JOURNAL OF CLINICAL AND LABORATORY INVESTIGATION, BLACKWELL SCIENTIFIC PUBLICATIONS, OXFORD, GB, Bd. 58, Nr. 3, Mai 1998 (1998-05), Seiten 205-216, XP000892355 ISSN: 0085-591X
- MORGENTHALER NILS G ET AL: "Immunoluminometric assay for the midregion of pro-atrial natriuretic peptide in human plasma." CLINICAL CHEMISTRY. UNITED STATES JAN 2004, Bd. 50, Nr. 1, Januar 2004 (2004-01), Seiten 234-236, XP008028257 ISSN: 0009-9147

### SEQUENCE LISTING

<110> B.R.A.H.M.S Aktiengesellschaft
<120> Sandwich-Immunoassay zur Bestimmung von pro-ANP Teilpeptiden
<130> 3901 PCT
<140>
   <141>
<150> 10254149.3
   <151> 2002-11-20
<160> 5
<170> PatentIn Ver. 2.1
<210> 1
   <211> 98
   <212> PRT
   <213> Artificial Sequence
<220>
   <223> Description of Artificial Sequence: Synthetic fragment
<400> 1
   Asn Pro Met Tyr Asn Ala Val Ser Asn Ala Asp Leu Met Asp Phe Lys
     1               5                  10                  15
   Asn Leu Leu Asp His Leu Glu Glu Lys Met Pro Leu Glu Asp Glu Val
                20                  25                  30
   Val Pro Pro Gln Val Leu Ser Glu Pro Asn Glu Glu Ala Gly Ala Ala
            35                  40                  45
   Leu Ser Pro Leu Pro Glu Val Pro Pro Trp Thr Gly Glu Val Ser Pro
        50                  55                  60
   Ala Gln Arg Asp Gly Gly Ala Leu Gly Arg Gly Pro Trp Asp Ser Ser
    65                  70                  75                  80
   Asp Arg Ser Ala Leu Leu Lys Ser Lys Leu Arg Ala Leu Leu Thr Ala
                    85                  90                  95
   Pro Arg
<210> 2
   <211> 128
   <212> PRT
   <213> Homo sapiens
<400> 2
   Asn Pro Met Tyr Asn Ala Val Ser Asn Ala Asp Leu Met Asp Phe Lys
   1 5 10 15
   Asn Leu Leu Asp His Leu Glu Glu Lys Met Pro Leu Glu Asp Glu Val
   20 25 30
   Val Pro Pro Gln Val Leu Ser Glu Pro Asn Glu Glu Ala Gly Ala Ala
   35 40 45
   Leu Ser Pro Leu Pro Glu Val Pro Pro Trp Thr Gly Glu Val Ser Pro
   50 55 60
   Ala Gln Arg Asp Gly Gly Ala Leu Gly Arg Gly Pro Trp Asp Ser Ser
   65 70 75 80
   Asp Arg Ser Ala Leu Leu Lys Ser Lys Leu Arg Ala Leu Leu Thr Ala
   85 90 95
   Pro Arg Ser Leu Arg Arg Ser Ser Cys Phe Gly Gly Arg Met Asp Arg
   100 105 110
   Ile Gly Ala Gln Ser Gly Leu Gly Cys Asn Ser Phe Arg Tyr Arg Arg
   115 120 125
<210> 3
   <211> 21
   <212> PRT
   <213> Artificial Sequence
<220>
   <223> Description of Artificial Sequence: Synthetic fragment
<400> 3
   Cys Pro Glu Val Pro Pro Trp Thr Gly Glu Val Ser Pro Ala Gln Arg
   1 5 10 15
   Asp Gly Gly Ala Leu
   20
<210> 4
   <211> 19
   <212> PRT
   <213> Artificial Sequence
<220>
   <223> Description of Artificial Sequence: Synthetic fragment
<400> 4
   Cys Gly Arg Gly Pro Trp Asp Ser Ser Asp Arg Ser Ala Leu Leu Lys
   1 5 10 15
   Ser Lys Leu
<210> 5
   <211> 12
   <212> PRT
   <213> Artificial Sequence
<220>
   <223> Description of Artificial Sequence: Synthetic fragment
<400> 5
   Cys Gly Arg Gly Pro Trp Asp Ser Ser Asp Arg Ser
   1 5 10

## Beschreibung

Die Erfindung betrifft Verfahren zur Bestimmung von proANP oder daraus gebildeten Teilpeptiden in biologischen Flüssigkeiten für Zwecke der medizinischen Diagnostik, Prognostik und therapiebegleitenden Verlaufskontrolle mittels eines Sandwich-Immunoassays (Zweiseiten-Immunoassays). Als "proANP-Teilpeptide" werden dabei im Rahmen der vorliegenden Anmeldung insbesondere das sogenannte NT-proANP oder proANP (1-98) und Teilpeptide, die eine midregionale Teilsequenz dieses NT-proANP enthalten, bezeichnet. Das erfindungsgemäße Verfahren wird insbesondere in der Kardialdiagnostik und Sepsisdiagnostik eingesetzt, wobei im weiteren der Begriff "Diagnostik" normalerweise als vereinfachender Oberbegriff verwendet wird, der auch Prognostik/Frühprognostik und therapiebegleitende Verlaufskontrolle einschließen soll.

Es ist seit langem bekannt, daß im Rahmen von Herzerkrankungen, insbesondere in den verschiedenen Phasen von Herzinsuffizienz sowie nach Myocardinfarkten, das als "atriales natriuretisches Peptid" (ANP, gelegentlich auch als atrialer natriuretischer Faktor, ANF, bezeichnet) bezeichnete Hormon in erhöhten Mengen ausgeschüttet wird, das aufgrund zahlreicher physiologischer Wirkungen wie Natriurese, Vasodilatation, Hemmung der Renin- und Aldosteronsekretion eine wichtige Rolle in der Homöostase des Wasserhaushalts und des Blutdrucks spielt. Als wichtigster Stimmulus für die ANP-Sekretion bei kardialen Erkrankungen wird die Vorhofdehnung angesehen.

Als das eigentliche Hormon ANP wird dabei üblicherweise ein 28-Aminosäuren umfassendes Peptid (99-126) vom C-terminalen Abschnitt eines 128 Aminosäuren umfassenden Prohormons (pro-ANP; SEQ ID NO:2) bezeichnet. Bei der Freisetzung des ANP aus dem Prohormon proANP wird, neben einer Abspaltung von zwei Aminosäuren (127/128) von dessen C-Terminus, in äquimolarer Menge auch das verbleibende größere Teilpeptid des proANP, das aus 98 Aminosäuren bestehende N-terminale proANP (NT-proANP; proANP (1-98)) in die Zirkulation abgegeben. Da dieses NT-proANP eine deutlich höhere Halbwertszeit bzw. Stabilität als ANP besitzt, kann NT-proANP als Laborparameter für die Diagnostik, Verlaufs- und Therapiekontrolle bei kardialen Erkrankungen genutzt werden. Zur Vertiefung wird hiermit verwiesen auf Lothar Thomas (Herausgeber), Labor und Diagnose, 5. erweiterte Auflage, Unterkapitel 2.14 des Kapitels 2., Kardiale Diagnostik, Seiten 116-118, und die darin zitierte Literatur.

Sowohl zur Bestimmung von ANP selbst als auch zur Bestimmung von NT-proANP in biologischen Flüssigkeiten (Serum, Plasma, Urin) wurden in der Vergangenheit verschiedene Immunoassays entwickelt und in der klinischen Forschung und Praxis eingesetzt. Der überwiegende Teil derartiger Immunoassays zur ANP- bzw. NT-proANP-Bestimmung beruht auf dem bekannten Prinzip kompetitiver Immunoassays, deren bekanntester Vertreter wiederum der Radioimmunoassay (RIA) ist. Kompetitive Immunoassays zur Bestimmunge von proANP bzw. ANP werden beispielsweise beschrieben bzw. verwendet in den Literaturstellen 1. bis 13. und 22. (RIA) sowie 14. und 15. (EIA) der beigefügten Literaturliste.

Kompetitive Immunoassays vom Typ RIA oder EIA können jedoch eine Reihe von Nachteilen aufweisen, die die praktische Handhabung und die Meßgenauigkeit betreffen, wobei als besondere Nachteile aufzuzählen sind eine nicht-optimale Sensitivität, eine mangelnde Robustheit, eine größere Anfälligkeit für Kreuzreaktionen und häufig auch lange Meßzeiten. Da ferner bei kompetitiven Immunoassays zur Bestimmung von Peptiden (die im.Immunoassay Analyten/Reagenzien vom Antigentyp darstellen) so vorgegangen wird, daß man mit einem Antikörper (polyklonal oder monoklonal) arbeitet, der spezifisch eine bestimmte Peptid-Teilsequenz erkennt, die gleichzeitig im Analyten und dem als Kompetitor bzw. für die Markierung verwendeten Immunreagens vorhanden ist, erkennen die üblichen kompetitiven Assays, als "Immunreaktivität", nur die Anwesenheit der jeweiligen Peptid-Teilsequenzen, ohne grundsätzlich zu unterscheiden, ob diese Sequenz Teil eines kürzeren oder längeren Peptids ist. Verwendet man allerdings verschiedene derartige kompetitive Immunoassays mit Antikörpern, die an verschiedene Teilbereiche einer bekannten Sequenz eines längeren Polypeptids binden, kann man aus der Parallelität bzw. Verschiedenheit der erhaltenen Meßergebnisse indirekt ableiten, ob mit den verschiedenen kompetitiven Immunoassays jeweils die gleichen Peptide gemessen wurden, oder ob die erkannten Peptid-Sequenzen Teile unterschiedlicher molekularer Peptidspezies waren, z.B. von Abbauprodukten. Verschiedene Abbauprodukte können dabei einerseits das Ergebnis verschiedener konkurrierender Abbaureaktionen sein. Jedoch auch wenn nur ein einziger Abbauweg beschritten wird, und alle Abbauprodukte aus ein und demselben Vorläuferpeptid stammen, können unterschiedliche Konzentrationen verschiedener Teilpeptide und Fragmente beobachtet werden. Enthalten die vermessenen Proben nämlich Abbauprodukte, die mit unterschiedlicher Geschwindigkeit gebildet werden oder als solche eine unterschiedliche Stabilität (Lebensdauer) aufweisen, ermittelt man für die verschiedenen Spezies unterschiedliche Konzentrationen, auch wenn diese aufgrund ihrer Herkunft aus ein und demselben Peptid primär nur in äquimolaren Mengen entstehen können.

Indem man zur Bestimmung von NT-proANP mit Hilfe kompetitiver Immunoassays unterschiedliche Antikörper verwendete, die verschiedene Sequenzen des NT-proANP (proANP 1-98; SEQ ID NO:1) erkannten, wurde festgestellt, daß in biologischen Flüssigkeiten, insbesondere Blut oder auch Urin, verschiedene Peptide auftreten, die Abbauprodukten des NT-proANP (proANP 1-98) entsprechen. Insbesondere wurde festgestellt, daß aus NT-proANP Fragmente mit niedrigeren Molekulargewichten gebildet werden, insbesondere solche, denen aufgrund ihrer Immunreaktivität die Aminosäuresequenzen 1-30, 31-67 und 79-98 des proANP zugeordnet wurden (vergleiche beispielsweise die Literaturstellen 3., 6., 7., 8., 9., 13., 14. und 15. der beigefügten Literaturliste).

Kompetitive Immunoassays, die spezifisch die genannten Sequenzen erkennen, erkennen in gleicher Weise - wenn man einmal Einflüsse unterschiedlicher Aviditäten oder eventuelle konformative Einflüsse nicht berücksichtigt - stets auch das vollständige NT-proANP und unterscheiden daher nicht zwischen diesem und seinen jeweiligen Fragmenten.

Nicht-kompetitive Sandwich-Immunoassays (Zweiseiten-Immunoassays) haben gegenüber kompetitiven Immunoassays eine Reihe von Vorteilen, zu denen gehört, daß sie besser als Festphasenassays (heterogene Assays) ausgelegt werden können, in der Handhabbarkeit robuster sein können, Meßergebnisse mit einer höheren Sensititivät liefern können und sich auch besser für eine Automatisierung und Serienmessung eignen. Außerdem können sie im Vergleich mit kompetitiven Immunoassays, die mit nur einem Typ von Antikörper arbeiten, auch etwas andere Aussagen liefern, indem Sandwich-Immunoassays nur solche Moleküle bzw. Peptide erkennen, bei denen beide Bindungsstellen für die für die Sandwichbildung verwendeten Antikörper auf dem gleichen Molekül vorhanden sind. Befinden sich die Bindungsstellen z.B. auf unterschiedlichen Teilpeptiden (Abbauprodukten, Fragmenten), führt eine Bindung der Antikörper an solche Fragmente nicht zu einem für den kompletten "Sandwich" typischen Meßsignal.

Wegen der bekannten Vorteile von Sandwich-Immunoassays generell sowie wegen der Möglichkeit, selektiv nur das vollständige NT-proANP zu messen, ohne daß die Messung durch Abbauprodukte und Fragmente beeinflußt wird, wurden auch schon Sandwich-Immunoassays zur Bestimmung von NT-proANP beschrieben und in der klinischen Forschung und Praxis eingesetzt. So wird in der EP 721 105 B1 ein Sandwich-Immunoassay zur Bestimmung von proANP beschrieben, und zwar insbesondere im Rahmen der Diagnostik kardialer Erkrankungen und von chronischem Nierenversagen, bei dem zwei monoklonale Antikörper verwendet werden, von denen einer an die Aminosäuren 1-25 des proANP (vgl. EP 350 218 B1) und der andere an die Aminosäuren 43-66 des proANP bindet. Mit einem solchen Sandwich-Immunoassay werden nur solche proANP-Teilpeptide erfaßt bzw. neben proANP(1-98) mit erfaßt, die die ersten 25 Aminosäuren der proANP-Sequenz enthalten.

Ein diesbezüglich ähnlicher weiterer Sandwich-Immunoassay ist beschrieben in der als Nr. 18 der beigefügten Literaturliste aufgeführten Veröffentlichung (Stridsberg). Auch dieser Sandwich-Immunoassay arbeitet mit zwei monoklonalen Antikörpern, von denen der eine an die Aminosäuren 1-30 des proANP bindet, während der ändere an die Aminosäuren 79-98 bindet. Aufgrund der Wahl der Bindungsstellen an den Enden des proANP (1-98) ist davon auszugehen, daß man mit diesem Sandwich-Immunoassay nur das intakte proANP (1-98) erfaßt.

In der WO 00/19207 wird ferner ein Verfahren zur Bestimmung von proANP 1-98 beschrieben, bei dem man mit zwei von drei polyklonalen Antikörpern arbeitet, die an die Aminosäuresequenzen 8-27, 31-64 bzw. 79-98 der Sequenz des proANP (1-98) binden. Der von der Anmelderin der WO 00/19207 kommerziell angebotene Assay zur Bestimmung von proANP (1-98) ist ein Enzym-Immunoassay, bei dem mit einem Paar von affinitätsgereinigten polyklonalen Antikörpern vom Schaf gearbeitet wird, von denen der immobilisiert eingesetzte Antikörper die Aminosäuren 10-19 des proANP erkennt, während zur Detektion ein zweiter polyklonaler Antikörper verwendet wird, der die Aminosäuren (85-90) erkennt (vgl. Arbeitsanleitung zum Sandwichassay proANP (1-98) der Fa. BIOMEDICA; A-1210 Wien). Auch dieser Assay erfaßt somit nur solche Peptidspezies, die die Endabschnitte des vollständigen proANP (1-98) enthalten, d.h. das vollständige NT-proANP.

Alle kompetitiv oder nach dem Sandwich-Prinzip arbeitenden Assays des Standes der Technik wurden im wesentlichen als Assays entwickelt, die für die kardiale Diagnostik bestimmt waren bzw. im Rahmen der kardialen Diagnostik verwendet wurden, wobei als weitere diagnostische Einsatzmöglichkeit außerdem noch chronisclies Nierenversagen erwähnt wurde (vgl. EP 721 105 B1 und Literaturstelle 29 der beigefügten Literaturliste).

In der WO 00/22439 der Anmelderin wird erstmals beschrieben, daß signifikant erhöhte Konzentrationen an proANP auch in Seren bzw. Plasmen von Sepsis-Patienten auftreten. Zur Bestimmung von proANP im Rahmen der beschriebenen orientierenden Versuche wurde ein im Handel erhältlicher RIA-Kit verwendet, bei dem nach dem kompetitiven Meßprinzip die Bindung an einen Antikörper bestimmt wurde, der die Aminosäure 26-55 des sogenannten preproANP erkannte, das gegenüber dem proANP am N-Terminus.um ein Signalpeptid aus 25 Aminosäuren verlängert ist. Die bei den in der WO 00/22439 beschriebenen Bestimmungen nachgewiesene(n) Peptidspezies wurde(n) nicht näher charakterisiert. Insbesondere wurde nicht untersucht, ob die erkannte Spezies ein kürzeres Teilpeptide vom N-Terminus des proANP war, ob sie das vollständige NT-proANP war, oder ob, in Analogie zu dem bei Sepsis beobachteten erhöhten Auftreten des bei Gesunden nicht nachweisbaren Procalcitonins in der Zirkulation, eine längere Spezies gemessen wurde, die das gesamte proANP (1-128) war oder wenigstens wesentliche Teile davon umfaßte.

Aus den Untersuchungen zum Auftreten des inzwischen als Sepsis- und Entzündungsmarker etablierten Procalcitonins (vgl. EP 656 121 B1 bzw. US 5 639 615 oder z.B. eine Übersichtsarbeit über spätere Erkenntnisse von W. Karzai et al. in Infection, Vol.25 (1997), S.329-334; DE 101 19 804 A1 sowie eine Reihe von weiteren, im Zeitpunkt der Anmeldung der vorliegenden Patentanmeldung noch nicht veröffentlichten Patentanmeldungen zum Thema Sepsisdiagnostik der Anmelderin) ist es bekannt, daß bei Sepsis, die als systemische Infektion oder systemisches Entzündungsgeschehen angesehen werden kann, viele physiologische Vorgänge, insbesondere auch zahlreiche Enzymaktivitäten, in stark veränderter Form ablaufen, was dazu führt, daß bei Sepsis in der Zirkulation zahlreiche Biomoleküle gefunden werden, die bei Gesunden nicht nachweisbar sind und die auch im Rahmen anderer Krankheitsgeschehen nicht oder nicht in dieser Form gebildet werden. So ist beispielsweise Procalcitonin normalerweise ein Vorläufer des Hormons Calcitonin, der nicht in die Zirkulation gelangt. Im Falle von Sepsis werden jedoch stark erhöhte Serum- und Plasma-Konzentrationen an Procalcitonin festgestellt, ohne daß gleichzeitig auch erhöhte Calcitoninkonzentrationen beobachtbar sind. Vor dem Hintergrund derartiger Erkenntnisse verbieten sich dann, wenn bei Sepsis Nachweisverfahren auf Biomarker ansprechen, die an sich aus anderen Zusammenhängen bereits bekannt sind, schnelle Analogieinterpretationen der labormedizinischer Befunden. Man muß immer damit rechnen, daß bei Sepsis andere molekulare Spezies in die Zirkulation gelangt sind, und andere proteolytische Abbaumechanismen aktiviert wurden, als im Falle anderer, insbesondere nicht infektiöser und nicht entzündlicher Erkrankungen.

In jüngster Zeit wurden einige weitere Arbeiten veröffentlicht, die den Nachweis von proANP bei Sepsis bestätigten (vergleiche Literaturstellen 24. und 25. der beigefügten Literaturliste). Das Aufteten des proANP wird dabei in erster Linie als Folge der bei Sepsis gestörten Herz-Kreislauf-Aktivität interpretiert. Es ist jedoch von Interesse, daß in anderen neueren Arbeiten (vergleiche 26: bis 28. der beigefügten Literaturliste) berichtet wird, daß das Hormon ANP auch wichtige Funktionen im Immunsystem auszuüben scheint, und daß ANP regulatorische Funktionen innerhalb der Reaktionskaskade eines Entzündungsgeschehens übernimmt.

Um die klinische Relevanz erhöhter proANP bzw. NT-proANP-Konzentrationen bei Sepsis gründlicher anhand zuverlässiger Meßdaten untersuchen zu können, sah es die Anmelderin als wünschenswert an, ein immundiagnostisches Bestimmungsverfahren zur Hand zu haben, das nicht mit den bekannten Nach-' teilen kompetitiver Bestimmungsverfahren behaftet ist, und das weniger als die bekannten Sandwich-Immunoassays davon abhängig ist, daß die in der Körperflüssigkeit der Patienten nachgewiesene Spezies das vollständige NT-proANP ist bzw. den vollständigen N-Terminus des NT-proANP aufweist.

Die Anmelderin entschloß sich daher, einen neuartigen Sandwich-Immunoassay zur Bestimmung von proANP bzw. sich davon ableitenden Teilpeptiden zu schaffen, der im Gegensatz zu den bisher bekannten derartigen Immunoassays nicht mit Antikörper arbeitet, die die Termini des NT-proANP erkennen, sondern mit Antikörpern, die beide in einem midregionalen Bereich des NT-proANPs binden. Überraschenderweise erwies sich der erhaltene Sandwich-Immunoassay nicht nur im Hinblick auf die proANP-Bestimmung im Rahmen der Sepsisdiagnostik als deutlich sensitiver als die vorbekannte kommerzielle Sandwich-Immunoassays, sondern zeigte auch auf dem Gebiet der Kardialdiagnostik eine deutlich verbesserte Sensitivität. Sensitivität ist dabei in der vorliegenden Anmeldung - wie üblich - so definiert, dass bei einer Sensitivität von 100% bei einer Bestimmung alle Kranken richtig als positiv erkannt werden; die Definition von Spezifität ist entsprechend, dass bei einer Spezifität von 100% alle Gesunden richtig als negativ bzw. keine Gesunden unrichtig als positiv erkannt werden.

Gegenstand der vorliegenden Erfindung ist daher gemäß Anspruch 1 ein Verfahren zur Bestimmung von proANP oder daraus gebildeten Teilpeptiden, die nicht das C-terminale ANP sind, in biologischen Flüssigkeiten zum Zwecke der medizinischen Diagnostik, Prognostik und therapiebegleitenden Verlaufskontrolle mittels eines Sandwich-Immunoassays (Zweiseiten-Immunoassays) unter Verwendung zweier Antikörper, die spezifisch an unterschiedliche Teilsequenzen des NT-proANP binden, das dadurch gekennzeichnet ist, daß beide Antikörper an Teilsequenzen im midregionalen Bereich des NT-proANP binden, der sich von etwa der Aminosäure 50 bis zur Aminosäure 90, insbesondere von der Aminosäure 53 bis 83, des NT-proANP erstreckt.

Die Antikörper können monoklonale und/oder polyklonale Antikörper sein, sind jedoch vorzugsweise affinätsgereinigte polyklonale Antikörper.

Besonders bevorzugt wird einer der Antikörper durch Immunisierung eines Tiers, insbesondere Schafs, mit einem Antigen erhalten, das eine synthetische Peptidsequenz enthält, die die Aminosäuren 53-72 des proANP sowie einen zusätzlichen Cysteinrest am N-Terminus aufweist, und der andere Antikörper wird entsprechend mit einem Antigen erhalten, das eine synthetische Peptidsequenz enthält, die die Aminosäuren 73-90 bzw. 73-83 des proANP mit einem zusätzlichen Cysteinrest am N-Terminus aufweist. Die unter Verwendung der genannten synthetischen Peptide, die gemeinsam einen lückenlosen midregionalen Abschnitt der proANP-Sequenz repräsentieren, erhaltenen Antikörper erkennen somit nur Bindungsstellen im Bereich der Aminosäuren 53-83 des NT-proANP, und in Form polyklonaler Antikörper können sie den genannten Bereich vollständig erfassen.

Bei einer bevorzugten Ausführungsform wird das Verfahren als heterogener Sandwich-Immunoassay durchgeführt, bei dem einer der Antikörper an eine beliebige Festphase, beispielsweise die Wände beschichteter Teströhrchen (z.B. aus Polystyrol; "Coated Tubes"; CT) oder an Mikrotiterplatten, zum Beispiel aus Polystyrol, oder an Partikel, beispielsweise Magnetpartikel immobilisiert ist, während der andere Antikörper einen Rest trägt, der ein direkt nachweisbares Label darstellt oder eine selektive Verknüpfung mit einem Label ermöglicht und der Detektion der gebildeten Sandwich-Strukturen dient. Auch eine zeitlich verzögerte bzw. nachträgliche Immobilisierung unter Verwendung geeigneter Festphasen ist möglich.

Grundsätzlich können alle in Assays der beschriebenen Art verwendbaren Markierungstechniken angewandt werden, zu denen Markierungen mit Radioisotopen, Enzymen, Fluoreszenz-, Chemolumineszenz- oder Biolumineszenz-Labeln und direkt optisch detektierbaren Farbmarkierungen, wie beispielsweise Goldatomen und Farbstoffteilchen, wie sie insbesondere für sog. Point-of-Care (POC) oder Schnelltests verwendet werden, gehören. Die beiden Antikörper können auch im Falle heterogener Sandwich-Immunoassays Teile eines Nachweissystems der nachfolgend im Zusammenhang mit homogenen Assays beschriebenen Art aufweisen.

Es liegt somit im Rahmen der vorliegenden Erfindung, das erfindungsgemäße Verfahren auch als Schnelltest auszugestalten.

Das erfindungsgemäße Verfahren kann ferner als homogenes Verfahren ausgestaltet werden, bei dem die aus den beiden Antikörpern und dem nachzuweisenden proANP bzw. proANP-Teilpeptid gebildeten Sandwichkomplexe in der flüssigen Phase suspendiert bleiben. In einem solchen Fall ist es bevorzugt, beide Antikörper mit Teilen eines Nachweissystems zu markieren, das dann, wenn beide Antikörper in einen einzigen Sandwich integriert werden, eine Signalerzeugung oder Signalauslösung ermöglicht. Derartige Techniken sind insbesondere als Fluoreszenzverstärkungs- oder Fluoreszenzlöschungs-Nachweisverfahren ausgestaltbar. Ein besonderes bevorzugtes derartiges Verfahren betrifft die Verwendung von paarweise einzusetzenden Nachweisreagenzien, wie sie beispielsweise beschrieben sind in US-A-4 822 733, EP-B1-180 492 oder EP-B1-539 477 und dem darin zitierten Stand der Technik. Sie ermöglichen eine Messung, die selektiv nur Reaktionsprodukte erfaßt, die beide Markierungskomponenten in einem einzigen Immunkomplex enthalten, direkt in der Reaktionsmischung. Als Beispiel ist auf die unter den Marken TRACE® (Time Resolved Amplified Cryptate Emission) bzw. KRYPTOR® angebotene Technologie zu verweisen, die die Lehren der o.g. Anmeldungen umsetzt.

Es zeigte sich überraschenderweise, daß erfindungsgemäß unter Verwendung zweier Antikörper, insbesondere zweier affinitätsgereinigter polyklonaler Antikörper, die beide im midregionalen Bereich, d.h. im Bereich der Aminosäuren von etwa 50 bis 90, insbesondere 53-83, der Sequenz des NT-proANP, binden, ein Assay mit einer gegenüber kommerziell erhältlichen Assays vom Sandwichtyp deutlich verbesserten Sensitivität erhalten wird. Diese Aussage gilt, wie nachfolgend gezeigt wird, nicht nur für die Sepsisdiagnostik, sondern auch auf dem Gebiet der Kardialdiagnostik.

Insbesondere letzterer Befund ist überraschend, da aus zahlreichen Veröffentlichungen bekannt war, daß beim weiteren proteolytischen Abbau des NT-proANP neben einem den Aminosäuren 1-30 des NT-proANP entsprechenden Teilpeptid auch zwei weitere Teilpeptide gebildet werden, die den Aminosäuren 31-67 sowie 79-98 entsprechen. Die im Rahmen des erfindungsgemäßen Verfahrens verwendeten Antikörper binden somit an verschiedene der bekannten NT-proANP-Abbauprodukte.

Es war in keiner Weise zu erwarten, daß mit den erfindungsgemäß zu verwendenden Antikörpern gegenüber anderen Sandwich-Verfahren mit Antikörpern, die an den Termini des NT-proANP (proANP 1-98) angreifen, eine Verbesserung erhalten werden könnte. Es sind in der Literatur keine Teilpeptide des NT-proANP beschrieben, die beide Bindungsstellen für die Antikörper gemäß der vorliegenden Erfindung enthalten, jedoch nicht den bei allen bekannten Sandwich-Immunoassays zur Bindung genutzten N-Terminus aufweisen. Ob die gemäß der vorliegenden Erfindung erhaltenen deutlich verbesserten Werte für die Assaysensitivität darauf zurückzuführen sind, daß der Abbau des NT-proANP nach einem bisher nicht beschriebenen Abbauschema erfolgt, oder ob die gewählten Bindungsstellen besser zugänglich sind und/oder eine festere selektivere Bindung oder reproduzierbare Mehrfachmarkierung ermöglichen und/oder durch die Bindung das Abbauerhalten in der Probe beeinflußt wird, soll offen bleiben. Die Interpretation der deutlichen, experimentell belegten verbesserten Meßergebnisse ist nicht Teil der vorliegende Erfindung.

Es wird davon ausgegangen, dass das erfindungsgemäße Bestimmungsverfahren besonders vorteilhaft auch im Rahmen einer sogenannten Multiparameterdiagnostik durchgeführt werden kann, und zwar sowohl auf dem Gebiet der Kardialdiagnostik als auch der Sepsisdiagnostik. Weitere dabei bestimmte Parameter sind beispielsweise die Kardialparameter BNP oder proBNP oder Sepsisparameter, die z.B. aus der Gruppe ausgewählt sind, die besteht aus Anti-Gangliosid-Antikörpern, den Proteinen Procalcitonin, CA 125, CA 19-9, S100B, S100A-Proteinen, LASP-1, löslichen Cytokeratin-Fragmenten, insbesondere CYFRA 21, TPS und/oder löslichen Cytokeratin-1-Fragmenten (sCY1F), den Peptiden Inflammin und CHP, anderen Peptid-Prohormonen, der Glycin-N-Acyltransferase (GNAT), der Carbamoylphosphat Synthetase 1 (CPS 1) und dem C-reaktiven Protein (CRP) oder Fragmenten davon. Bei den genannten Multiparameter-Bestimmungen ist vorgesehen, die Messergebnisse für mehrere Parameter gleichzeitig oder parallel zu bestimmen und z.B. mit Hilfe eines Computerprogramms, das auch diagnostisch signifikante Parameterkorrelationen nutzt, auszuwerten.

Nachfolgend wird die Erfindung durch eine Beschreibung der Herstellung der Assay-Komponenten, der Durchführung einer bevorzugten Ausführungsform des Assays und der unter Verwendung des erfindungsgemäßen Assays erhaltenen Ergebnisse von proANP-Bestimmungen in EDTA-Plasmen von Kontrollpersonen und Herz- und Sepsispatienten näher erläutert.

Dabei wird auf Figuren Bezug genommen, die zeigen:
- Fig. 1: die Bestimmung von proANP in einem Kontrollkollektiv von Gesunden, einem Kollektiv von Sepsispatienten und einem Kollektiv von Angina Pectoris-Patienten mit Hilfe des erfindungsgemäßen proANP-Assays;
- Fig. 2: entsprechende Meßergebnisse für die gleichen Proben wie in Fig.1, wie sie mit einem kommerziell erhältlichen proANP-Sandwich-Assay erhalten wurden, bei dem zwei Antikörper verwendet werden, die an die Aminosäuren 10-19 bzw. 85-90 des proANP binden;
- Fig. 3: sogenannte ROC-Kurven (Receiver Operating Characteristic Plots), die die Sensivitäten und Spezifitäten der proANP-Bestimmung gemäß den Figuren 1 und 2 im Vergleich zeigen. Die Darstellung ermöglicht einen Vergleich der Wertigkeit der Bestimmungen mit den verglichenen Assays bei Sepsis. Bei ROC-Kurven kann die Fläche zwischen der betreffenden Kurve und einer unter einem Winkel von 45° verlaufenden Geraden ("area under the ROC function", AUC) als Kenngröße für die statistische Relevanz einer Bestimmung genommen werden.
- Fig. 4: eine der Figur 3 entsprechende Darstellung, die einen Vergleich der Wertigkeit der Bestimmungen mit den verglichenen Assays bei Angina pectoris ermöglicht.

### Experimenteller Teil

### Materialien und Methoden

Für die Antikörpergewinnungen wurden anfänglich zwei Abschnitte der bekannten Aminosäuresquenz des proANP (SEQ ID NO:2) bzw. des humanen NT-proANP (SEQ ID NO:1) ausgewählt, die deren Positionen 53-72 bzw. 73-90 entsprachen. Aufgrund der Ergebnisse eines Epitopmappings wurde später bei der endgültigen Antikörpergewinnung statt der Sequenz 73-90 eine C-terminal verkürzte Sequenz 73-83 verwendet. Alle Abschnitte wurden, ergänzt um einen N-terminalen Cysteinrest, als lösliche Peptide nach Standardverfahren chemisch synthetisiert, aufgereinigt, mittels Massenspektrometrie und Reversed Phase HPLC qualitätskontrolliert und in Aliquots lyophilisiert (die Arbeiten wurden im Auftrag der Anmelderin von der Firma JERINI AG, Berlin, Deutschland) durchgeführt. Die Aminosäuresequenzen der synthetischen Peptide sind wie folgt:
Peptid "MPCL21" (Positionen 53-72; SEQ ID NO:3):
   CPEVPPWTGEVSPAQRDGGAL
Peptid "SPCL19" (Positionen 73-90; SEQ ID NO:4):
   CGRGPWDSSDRSALLKSKL
Peptid "SPCL12" (Positionen 73-83; SEQ ID NO:5):
   CGRGPWDSSDRS

### Immunisierung

Für Immunisierungszwecke wurden die Peptide MPCL21 und SPCL 19 mittels MBS (m-Maleimidobenzoyl-N-hydroxysuccinimid Ester) an das Trägerprotein KLH (Keyhole limpet hemocyanin) konjugiert, wobei man der Arbeitsanleitung "NHS-Esters-Maleimid Crosslinkers" der Firma PIERCE, Rockford, IL, USA folgte.

Mit den erhaltenen Konjugaten wurden Schafe nach folgendem Schema immunisiert: Jedes Schaf erhielt initial 100 µg Konjugat (die Massenangaben sind bezogen auf den Peptid-Anteil des Konjugats) und anschließend in 4 Wochen-Intervallen jeweils weitere 50 µg Konjugat. Beginnend mit dem vierten Monat nach Immunisierungsbeginn wurden in 4 Wochen-Intervallen den Schafen 700 ml Blut abgenommen, und daraus wurde durch Zentrifugation Antiserum gewonnen.

Konjugationen, Immunisierungen und Gewinnung von Antiseren wurden im Auftrag der Anmelderin von der Firma MicroPharm, Carmarthenshire, UK, durchgeführt.

### Reinigung der Antikörper

In einem 1-Schritt Verfahren wurden aus den Antiseren, die ab dem vierten Monat nach der Immunisierung gewonnen worden waren, die Peptid-spezifischen Antikörper präpariert.

Dafür wurden zuerst die Peptide MPCL21 sowie SPCL19, später statt des letzteren etwas das kürzere Peptid SPCL12, entsprechend der Arbeitsanleitung des Herstellers ("SulfoLink Kit", Firma PIERCE, Rockford, IL, USA) an SulfoLink Gel gekoppelt. Dabei wurden zur Kopplung jeweils 5 mg Peptid pro 5 ml Gel eingesetzt.

Die Affinitätsreinigung von gegen die beiden Peptide MPCL21 und SPCL19 bzw. SPCL12 spezifischen Antikörpern aus den Schaf-Antiseren wurde wie folgt durchgeführt:

Die jeweiligen SulfoLink-Gele wurden in Trennsäulen gegeben.

Die erhaltenen Peptid-Säulen wurden zunächst 3 mal im Wechsel mit je 10 ml Elutionspuffer (50 mM Citronensäure, pH 2,2) und Bindungspuffer (100 mM Natriumphosphat, 0,1% Tween, pH 6,8) gewaschen. 100 ml der Antiseren wurden über 0,2 µm-Filter filtriert und mit dem jeweiligen Peptid-gekoppelten Gel versetzt, das zu diesem Zwecke quantitativ mit 10 ml Bindungspuffer aus der Säule, in der es gewaschen worden war, gespült wurde. Die Inkubation von Peptid-gekoppelten Gel mit den Antiseren erfolgte über Nacht bei Raumtemperatur in Schwenkkolben. Danach wurden die Ansätze quantitativ in Leersäulen (NAP 25, Pharmacia, entleert) überführt. Das ablaufende Material wurde verworfen. Anschließend wurde mit 250 ml Bindungspuffer (s.o.) proteinfrei gewaschen (der Proteingehalt des Wascheluats zeigte eine Absorption von 280 nm von weniger als 0,02). Auf die gewaschenen Säulen wurde nunmehr Elutionspuffer gegeben, und das Eluat wurd in Fraktionen à 1 ml gesammelt. Von jeder Fraktion wurde der Proteingehalt mittels der BCA-Methode (Bicinchoninic Acid; vgl. PIERCE Chemical Technical Library, www.piercenet.com) bestimmt. Die Fraktionen 4-7 wurden gepoolt, und bei der Proteinbestimmung der gepoolten Fraktionen mittels der BCA-Methode ergaben sich Ausbeuten von 15,5 mg für den anti-MPCL21-Antikörper und von 20,7 mg für den anti-SPCL12 Antikörper.

Bei einem Epitop-Mapping der auf die beschriebene Weise erhaltenen Antikörper wurde festgestellt, dass affinitätsgereinigte Antikörper, die mit dem Peptid SPCL19 erhalten wurden, nahezu ausschließlich an Epitope im Bereich der Aminosäuren 73-83, d.h. einem dem Peptid SPLC12 entsprechenden Abschnitt, banden. Daher wurde das Peptid SPLC19 bei der Affinitätsreinigung durch das Peptid SPCL12 ersetzt.

### Markierung

Zur Herstellung eines Detektionsantikörpers wurden 500 µl des wie beschrieben affinitätsgereinigten polyklonalen anti-MPCL21-Antikörpers über eine NAP-5 Gelfiltrationssäule (Pharmacia) in 1 ml 100 mM Kalium-Phosphatpuffer (pH 8,0) nach Arbeitsanleitung umgepuffert. Die Proteinkonzentrationsbestimmung der Antikörperlösung ergab einen Wert von 1,5 mg/ml.

Um diesen Antikörper mit einer Chemilumineszenzmarkierung zu versehen, wurden 132 µl der Antikörperlösung mit 10 µl MA70-Akridinium-NHS-Ester (1 mg/ml; Firma HOECHST Behring) versetzt und 15 min bei Raumtemperatur inkubiert. Dann wurden bei 358 µl 1 M Glycin zugesetzt, und es wurde weitere 10 min inkubiert. Danach wurde der Markierungsansatz über eine NAP-5 Gelfiltrationssäule (Pharmacia) in 1 ml Laufmittel A (1 mM Kaliumphosphat, 10% Methanol, 0,1% Lubrol, 0,1% Azid, pH 6,8) nach Arbeitsanleitung umgepuffert und dabei von niedermolekularen Bestandteilen befreit. Zur Abtrennung letzter Reste nicht an Antikörper gebundener Label-Bestandteile wurde eine HPLC an Hydroxylapatit durchgeführt (Säule: Knauer, 10 cm x 8 mm ID, Ser. Nr. LF230 Batch Nr. 1250000A, Hydroxyapatite Bio Rad 10 µm). Die Probe wurde aufgetragen und bei einer Flußrate von 1ml/min mittels eines linearen Gradienten Laufmittel A/Laufmittel B chromatographiert. Als Laufmittel B wurde dabei eingesetzt eine Lösung von 500 mM Kaliumphosphat, 10% Methanol, 0,1% Lubrol, 0,1% Azid, pH 6,8. Mit einem Durchflußphotometer wurden kontinuierlich die Wellenlängen 280 nm und 368 nm gemessen. Das Absorptionsverhältnis 368 nm/280 nm spiegelt den Markierungsgrad des Antikörpers wieder und betrug am Peak 0,16.

Die Antikörper enthaltenden Fraktionen, die bei ca. 40% Laufmittel B eluierten, wurden gesammelt und in 3 ml 100 mM Natriumphosphat, 150 mM NaCl, 5% Bovines Serum Albumin (BSA), 0,1% Natriumazid, pH 7,4 gesammelt.

Diese Lösung stellt ein Tracerkonzentrat dar, das bei den nachfolgend beschriebenen Bestimmungen eingesetzt wird.

### Kopplung

Zur Herstellung einer Festphase für das erfindungsgemäße Bestimmungsverfahren wurden bestrahlte 5 ml Polystyrolröhrchen (Firma Greiner) zuerst mit Esel anti-Schaf IgG Antikörpern (Firma Scantibodies) wie folgt beschichtet:

Der Antikörper wurde in 50 mM Natriumphosphat, pH 6,5, auf eine Konzentration von 16,7 µg/ml verdünnt. In jedes Röhrchen wurden 300 µl dieser Lösung pipettiert. Anschließend wurden die-Röhrchen für 20 h bei 22°C inkubiert. Die Lösung wurde abgesaugt, wonach jedes Röhrchen mit 4,2 ml 10 mM Natriumphosphat, 2% Karion FP, 0,3% BSA, pH 6,5 befüllt wurde. Nach 20 Stunden wurde die Lösung ebenfalls abgesaugt.

Der gereinigte Schaf-Antikörper, der an das Peptid SPCL12 band, wurde in 50 mM Natriumphosphat, 50 mM NaCl, 0,1% Natriumazid, 0,05% BSA, pH 7,8 auf eine Konzentration von 4,3 µg/ml verdünnt. In jedes Röhrchen wurden 300 µl dieser Lösung pipettiert. Anschließend wurden die Röhrchen 20h bei 22°C inkubiert, wonach die Lösung wieder abgesaugt wurde. Dann wurdes jedes Röhrchen mit 4,2 ml 10 mM Natriumphosphat, 2% Karion FP, 0,3% BSA, pH 6,5 befüllt. Nach 20 h wurde die Lösung abgesaugt. Anschließend wurden die Röhrchen in einem Vakuumtrockner getrocknet.

### Durchführung und Auswertung des erfindungsgemäßen Immunoassays

Für die Bestimmungen wurde ein Assaypuffer verwendet, der folgende Zusammensetzung aufwies:
100 mM Natriumphosphat, 150 mM NaCl, 5% BSA, 0,1% unspezifische Schaf IgG, 0,1% Natriumazid, pH 7,4.

Als Standardmaterial diente ein Extrakt eines E. coli-Stamms, der rekombinantes proANP (1-128) exprimierte (InVivo GmbH, Hennigsdorf, Deutschland). Dieser Extrakt wurde seriell in Pferdenormalserum (Firma SIGMA) zur Herstellung von Standardlösungen verdünnt. Den so hergestellten Standardlösungen wurden arbiträre ProANP-Konzentrationen, entsprechend den eingesetzten Verdünnungen des Standardmaterials, zugeordnet.

Als Proben wurden EDTA-Plasmen von augenscheinlich Gesunden, von Sepsis-Patienten und von Patienten mit Angina Pectoris vermessen.

Dazu wurde im ersten Schritt der Bestimmung in die beschichteten Teströhrchen 200 µl Assaypuffer sowie in Doppelbestimmungen 20 µl der Standards bzw. Proben pipettiert. Anschließend wurde über Nacht bei 22°C unter Schütteln inkubiert. Dann wurde 4x mit je 1ml Waschlösung (0,1% Tween 20) pro Röhrchen gewaschen, und man ließ die Röhrchen abtropfen. Dann wurden in jedes Röhrchen 200 µl einer 1:1.000-Verdünnung des obigen Tracerkonzentrats in Assaypuffer pipettiert. Es wurde 2 h bei 22°C unter Schütteln inkubiert. Dann wurde 4x mit je 1 ml der obigen Waschlösung pro Röhrchen gewaschen, man ließ die Röhrchen abtropfen und per Maß abschließend die an das Röhrchen gebundene Chemilumineszenz in einem Luminometer (Firma BERTHOLD, LB952T; Basisreagenzien BRAHMS AG) vermessen. Unter Verwendung der Software MultiCalc (Spline Fit) wurden die proANP-Konzentrationen der Proben an der Standardkurve abgelesen.

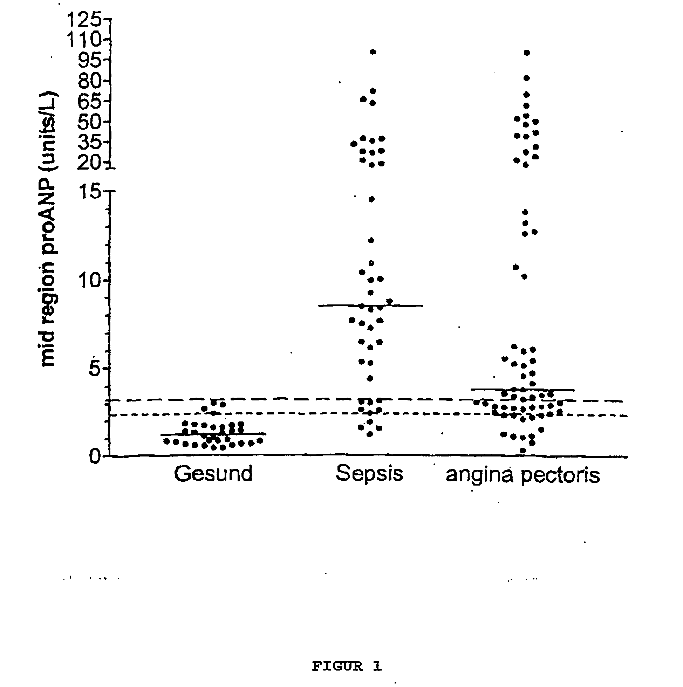

Die für die proANP-Bestimmungen in EDTA-Plasmen von Gesunden (Kontrollproben), Sepsispatienten und Patienten mit Angina Pectoris mit dem erfindungsgemäßen Assay erhaltenen Ergebnisse sind in Figur 1 gezeigt. Als "Cut-Offs" dienen zwei Schwellenwerte, die so gewählt wurden, dass 100% aller Gesunden bzw. 90% aller Gesunden als negativ erfaßt werden (Meßwerte unter dem als Cut-Off gewählten Schwellenwert aufweisen).

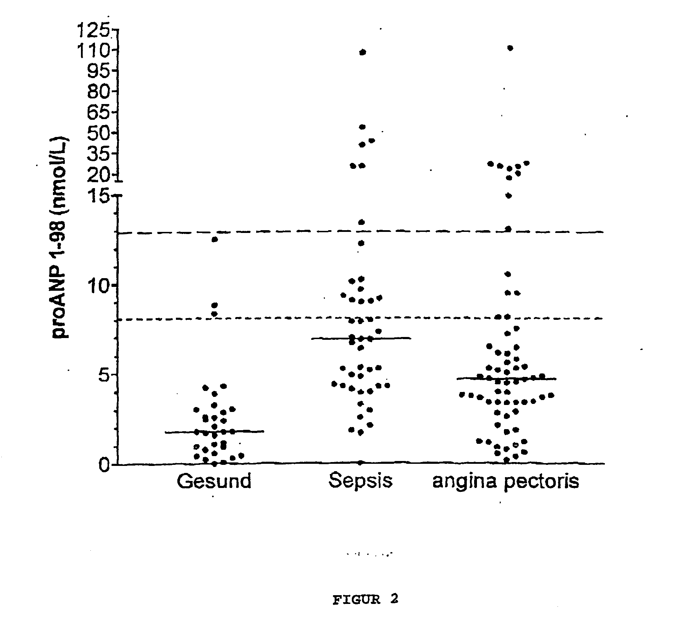

Nach der Arbeitsanleitung des Herstellers (Arbeitsanleitung zum Sandwichassay proANP (1-98) der Fa. BIOMEDICA; A-1210 Wien) wurden die gleichen Proben außerdem mit einem kommerziell erhältlichen Sandwich-Assay gemessen. Die Ergebnisse sind in Figur 2 dargestellt, wobei die Cut-Offs wie oben festgelegt wurden.

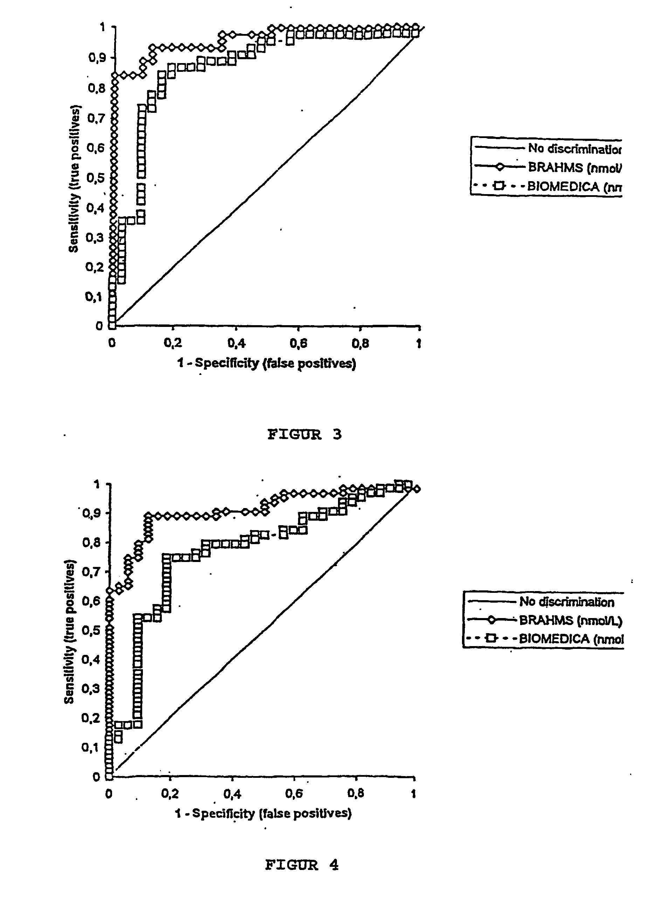

In Figur 3 werden die mit den beiden Assays erhaltenen Ergebnisse der Bestimmungen bei Sepsispatienten unter Verwendung von ROC-Kurven verglichen, und in Figur 4 die Ergebnisse bei Herzpatienten. Orientiert man sich jeweils an den direkt optisch entnehmbaren AUC-Werten (vgl. Erläuterungen bei Figur 3) der jeweligen ROC-Kurven, ist sofort zu erkennen, dass das erfindungsgemäße Verfahren Messwerte liefert, die bezüglich ihrer statistischen Relevanz deutlich überlegen sind.

### LITERATURLISTE

1. Hiroshi Itoh et al., Peptides derived from atrial natriuretic polypeptide precursor in human and monkey brains; Journal of Hypertension 1988, 6 (suppl 4) S309-S319;
2. L.Meleagros et al., ProAtrial Natriuretic Peptide (1-98): The Circulating Cardiodilatin in Man; Peptides, Vol. 10, 545-550, 1989
3. M.G.Buckley et al., Concentration of N-terminal ProANP in human plasma: evidence for ProANP (1-98) as the circulating form; Clinica Chimica Acta, 191 (1990) 1-14
4. Amir Lerman et al., Circulating N-terminal atrial natriuretic peptide as a marker for symptomless left-ventricular dysfunction; Lancet 1993; 341:1105-09, 1993
5. John G F Cleland et al., Stability of plasma concentrations of N and C terminal atrial natriuretic peptides at room temperature; Heart 1996; 75:410-413;
6. J.B. Hansen et al., Proatrial natriuretic polypeptide (31-67) in healthy individuals; day-to-day variation and influence of sex and age; Clin.Lab.Invest. 1995; 55:447-452
7. Rose M. Overton et al., Processing of Long-Acting Natriuretic Peptide and Vessel Dilator in Human Plasma and Serum; Peptides, Vol.17, 1155-1162, 1996
8. Sreedevi Daggubati et al., Adrenomedullin, endothelin, neuropeptide Y, atrial, brain, and C-natriuretic pro-hormone peptides compared as early heart failure indicators; Cardiovascular Research 36 (1997) 246-255
9. E.F. Macaulay Hunter et al., Analysis of peptides derived from Pro Atrial Natriuretic Peptide that circulate in man and increase in heart disease; Scand J Clin Invest 1998; 58: 205-216;
10. Martin G. Buckley et al., Cardiac peptide stability, aprotinin and room temperature: importance for assessing cardiac function in clinical practice; Clinical Science (1999) 97: 689-695
11. Randi Klinge et al., N-terminal proatrial natriuretic peptide in angina pectoris: impact of revascularization by angioplasty; International Journal of Cardiology 68 (1999), 1-8
12. H. Holmström et al., Plasma levels of N-terminal proatrial natriuretic peptide in children are dependent on renal function and age; Scand J Clin Invest 2000; 60: 149-160
13. Martina Franz et al., N-terminal fragments of the proatrial natriuretic peptide in patients before and after hemodialysis treatment; Kidney International, Vol. 58 (2000), 374-383
14. Engelbert Hartter et al., Enzyme Immunoassays for Fragments (Epitopes) of Human Proatrial Natriuretic Peptides; Clin Chem Lab Med 2000; 38(1):27-32
15. Enrico Cappelin et al., Plasma atrial natriuretic peptide (ANP) fragments proANP (1-30) and proANP (31-67) measurements in chronic heart failure; a useful index for heart transplantation? Clinica Chimica Acta 310 (2001) 49-52
16. Tomas Jernberg et al., Usefulness of Plasma N-Terminal Proatrial Natriuretic Peptide (proANP) as an Early Predictor of Outcome in Unstable Angina Pectoris or Non-ST-Elevation Acute Myocardial Infarction; The American Journal of Cardiology, Vol. 89, (2002), 64-66
17. Arbeitsanleitung zum Sandwichassay proANP (1-98) der Fa. BIOMEDICA; A-1210 Wien, Divischgasse 4 (2 affinitätsgereinigte polyklonale Ab gegen AS 10-19 und 85-90)
18. Mats Stridsberg et al., A Two-Site Delfia Immunoassay for Measurement of the N-terminal Peptide of pro-Atrial Natriuretic Peptide (nANP); in: Uppsala J.Med.Sci. 102; 99-108, 1997 (2 mAb gegen 1-30 und 79-98)
19. Lothar Thomas (Herausgeber), Labor und Diagnose, TH-Books, Frankfurt/Man, 5.erw.Auflage 2000, Seiten 114-118;
20. Chieko Mitaka et al., Plasma alpha-atrial natriuretic peptide concentration in acute respiratory failure associated with sepsis: Preliminary study; Critical Care Medicine, 1990, Vol. 18:1201-1203
21. Chieko Mitaka et al., Endothelin-1 and atrial natriuretic peptide in septic shock; American Heart Journal 1993; Vol. 126, No.2, 466-468
22. Fumiaki Marumo et al., A Highly Sensitive Radioimmunoassay of Atrial Natriuretic Peptide (ANP) in Human Plasma and Urine; Biochem.Biophys.Res.Commun. 137:231-236 (1985)
23. Koichi Aiura et al., Circulating concentrations and physiologic role of atrial natriuretic peptide during endotoxic shock in the rat; Critical Care Medicine 1995; 23:1889-1906
24. Mazul-Sunko et al., Pro-atrial natriuretic peptide hormone from the right atria is correlated with cardia depression in septic patients; J.Endocrinol.Invest 24; R22-R24, 2001
25. Koen J. Hartemink et al., alpha-Atrial natriuretic peptide, cyclic guanosine monophosphate, and endothelin in plasma as markers of myocardial depression in human septic shock; Critical Care Medicine 2001; 29:80-87
26. A K Kiemer et al., The atrial natriuretic peptide regulates the production of inflammatory mediators in macrophages; Ann Rheum Dis 2001; 60:iii68-iii70
27. Alexandra K. Kiemer et al., The Atrial Natriuretic Peptide as a Regulator of Kupffer Cell Function; Shock, Vol.17, S.365-371, 2002
28. Alexandra K. Kiemer et al., Induction of IκB: atrial natriuretic peptide as a regulator of the NF-κB pathway; Biochem.Biophys.Res.Commun. 295 (2002) 1068-1076
29. Buckley MG et al., N-terminal pro atrial natriuretic peptide in human plasma; Am.J.Hypertens. 1990 Dec.; 3 (12Pt 1): 933-935.

## Patentansprüche

1. In vitro-Verfahren zur Bestimmung von proANP gemäß SEQ ID NO:2 oder daraus gebildeten Teilpeptiden, die nicht das C-terminale ANP aus den Aminosäuren 99-126 des proANP gemäß SEQ ID NO:2 sind, in biologischen Flüssigkeiten für Zwecke der medizinischen Diagnostik, Prognostik und therapiebegleitenden Verlaufskontrolle mittels eines Sandwich-Immunoassays unter Verwendung zweier Antikörper, die spezifisch an unterschiedliche Teilsequenzen des N-terminalen proANP gemäß SEQ ID NO:1 binden, **dadurch gekennzeichnet, dass** beide Antikörper an Teilsequenzen im midregionalen Bereich des NT-proANP binden, der sich von der Aminosäure 50 bis zur Aminosäure 90 des NT-proANP gemäß SEQ ID NO:1 erstreckt.

2. Verfahren nach Anspruch 1, **dadurch gekennzeichnet, dass** beide,Antikörper an Teilsequenzen in einem midregionalen Bereich des NT-proANP binden, der sich von der Aminosäure 53 bis zur Aminosäure 83 des NT-proANP erstreckt.

3. Verfahren nach Anspruch 1 oder 2, **dadurch** gekennzeichent, dass die Antikörper monoklonale und/oder polyklonale Antikörper sind.

4. Verfahren nach einem der Ansprüche 1 bis 3, **dadurch gekennzeichnet, dass** beide Antikörper affinitätsgereinigte polyklonale Antikörper sind.

5. Verfahren nach einem der Ansprüche 1 bis 4, **dadurch gekennzeichnet, dass** einer der Antikörper durch Immunisierung eines Tiers mit einem Antigen erhalten wird, das eine synthetischen Peptidsequenz enthält, die die Aminosäuren 53-72 gemäß SEQ ID NO:3 des proANP umfaßt, und der andere der Antikörper durch Immunisierung mit einem Antigen erhalten wird, das eine synthetische Peptidsequenz enthält, die die Aminosäuren 73-90 gemäß SEQ ID NO:4 des proANP umfaßt.

6. Verfahren nach einem der Ansprüche 1 bis 5, **dadurch gekennzeichnet, dass** einer der Antikörper markiert ist und der andere Antikörper an eine Festphase gebunden ist oder selektiv an eine Festphase gebunden werden kann.

7. Verfahren nach einem der Ansprüche 1 bis 5, **dadurch gekennzeichnet, dass** sowohl der erste als auch der zweite Antikörper in der flüssigen Reaktionsmischung dispergiert vorliegen und daß an den ersten Antikörper eine erste Markierungskomponente gebunden ist, die Teil eines auf Fluoreszenz- oder Chemilumineszenz-Löschung oder -Verstärkung beruhenden Markierungssystems ist, und daß die zweite Markierungskomponente dieses Markierungssystems an den zweiten Antikörper gebunden ist, so dass nach Bindung beider Antikörper an das nachzuweisende proANP ein messbares Signal erzeugt wird, das eine Detektion der gebildeten Sandwichkomplexe in der Messlösung ermöglicht.

8. Verfahren nach Anspruch 7, **dadurch gekennzeichnet, dass** das Markierungssystem Seltenerdkrypate oder -chelate in Kombination mit einem Fluoreszenz- oder Chemilumineszenz-Farbstoff, insbesondere von Cyanintyp, umfaßt.

9. Verfahren nach einem der Ansprüche 1 bis 8, **dadurch gekennzeichnet, dass** es auf dem Gebiet der Kardiäldiagnose angewandt wird..

10. Verfahren nach Anspruch 9, **dadurch gekennzeichnet, dass** es im Rahmen einer Multiparameter-Bestimmung durchgeführt wird, bei der gleichzeitig weitere für die Kardialdiagnostik relevante Parameter, insbesondere BNP und proBNP, bestimmt werden.

11. Verfahren nach einem der Ansprüche 1 bis 8, **dadurch gekennzeichnet, dass** es zur Diagnose, für die Bestimmung.der Schweregrads und Prognostik sowie zur verlaufsbegleitenden Therapiekontrolle von Sepsis angewandt wird.

12. Verfahren nach Anspruch 11, **dadurch gekennzeichnet, dass** es im Rahmen einer Multiparameter-Bestimmung durchgeführt wird, bei der gleichzeitig mindestens ein weiterer für die Sepsisdiagnostik relevante Parameter bestimmt wird.

13. Verfahren nach Anspruch 12, **dadurch gekennzeichnet, dass** der oder die weitere(n) für die Sepsisdiagnostik relevante(n) Parameter aus der Gruppe ausgewählt sind, die besteht aus Anti-Gangliosid-Antikörpern, den Proteinen Procalcitonin, CA 125, CA 19-9, S100B, S100A-Proteinen, LASP-1, löslichen Cytokeratin-Fragmenten, insbesondere CYFRA 21, TPS und/oder löslichen Cytokeratin-1-Fragmenten (sCY1F), den Peptiden Inflammin und CHP, anderen Peptid-Prohormonen, der Glycin-N-Acyltransferase (GNAT), der Carbamoylphosphat Synthetase 1 (CPS 1) und dem C-reaktiven Protein (CRP) oder Fragmenten davon.

## Claims

1. *In vitro* method for determining proANP according to SEQ ID NO:2 or partial peptides formed therefrom, which are not the C-terminal ANP consisting of the amino acids 99-125 of proANP according to SEQ ID NO:2, in biological fluids for medical diagnosis, prognosis and therapy-accompanying monitoring by means of a sandwich immunoassay using two antibodies which bind specifically to different partial sequences of N-terminal proANP according to SEQ ID NO:1, **characterized in that** both antibodies bind to partial sequences in the midregional region of NT-proANP which extends from amino acid 50 to amino acid 90 of NT-proANP according to SEQ ID NO:1.

2. Method according to Claim 1, **characterized in that** both antibodies bind to partial sequences in a midregional region of NT-proANP which extends from amino acid 53 to amino acid 83 of NT-proANP.

3. Method according to Claim 1 or 2, **characterized in that** the antibodies are monoclonal and/or polyclonal antibodies.

4. Method according to any of Claims 1 to 3, **characterized in that** both antibodies are affinity-purified polyclonal antibodies.

5. Method according to any of Claims 1 to 4, **characterized in that** one of the antibodies is obtained by immunization of an animal with an antigen which contains a synthetic peptide sequence which comprises the amino acids 53-72 according to SEQ ID NO:3 of proANP and the other antibody is obtained by immunization with an antigen which contains a synthetic peptide sequence which comprises the amino acids 73-90 according to SEQ ID NO:4 of proANP.

6. Method according to any of Claims 1 to 5, **characterized in that** one of the antibodies is labelled and the other antibody is bound to a solid phase or can be selectively bound to a solid phase.

7. Method according to any of Claims 1 to 5, **characterized in that** both the first and the second antibody are present in dispersed form in the liquid reaction mixture and that a first labelling component which is part of a labelling system based on fluorescence or chemiluminescence extinction or amplification is bound to the first antibody, and that the second labelling component of this labelling system is bound to the second antibody so that, after binding of both antibodies to the proANP to be detected, a measurable signal is generated which permits detection of the resulting sandwich complexes in the measuring solution.

8. Method according to Claim 7, **characterized in that** the labelling system comprises rare earth cryptates or chelates in combination with a fluorescent or chemiluminescent dye, in particular of the cyanine type.

9. Method according to any of Claims 1 to 8, **characterized in that** it is used in the field of cardiac diagnosis.

10. Method according to Claim 9, **characterized in that** it is carried out in a multiparameter determination in which further parameters relevant to cardiac diagnosis, in particular BNP and proBNP, are simultaneously determined.

11. Method according to any of Claims 1 to 8, **characterized in that** it is used for diagnosis, for determination of the severity and prognosis and for accompanying therapy control of sepsis.

12. Method according to Claim 11, **characterized in that** it is carried out in a multiparameter determination in which at least one further parameter relevant to sepsis diagnosis is simultaneously determined.

13. Method according to Claim 12, **characterized in that** the parameter, or the further parameters, relevant to sepsis diagnosis is or are selected from the group consisting of anti-ganglioside antibodies, the proteins procalcitonin, CA 125, CA 19-9, S100B, S100A proteins, LASP-1, soluble cytokeratin fragments, in particular CYFRA 21, TPS and/or soluble cytokeratin-1 fragments (sCY1F), the peptides inflammin and CHP, other peptide prohormones, glycine N-acyltransferase (GNAT), carbamoylphosphate synthetase 1 (CPS 1) and the C-reactive protein (CRP) or fragments thereof.

## Revendications

1. Procédé pour déterminer in vitro dans des liquides biologiques la proANP de SEQ ID NO:2 ou de peptides partiels formés à partir de cette séquence, qui ne sont pas l'ANP de la terminaison C constituée des acides aminés 99 à 126 de la proANP selon SEQ ID NO:2, en vue du diagnostic médical, du pronostic et du contrôle d'accompagnement d'une thérapie, au moyen d'une immunodétermination en sandwich par recours à deux anticorps qui se lient spécifiquement à des séquences partielles différentes de la proANP de la terminaison N de SEQ ID NO:1, **caractérisé en ce que** les deux anticorps se lient à des parties de séquence situées dans la partie médiane de la NT-proANP qui s'étend entre l'acide aminé 50 et l'acide aminé 90 de la NT-proANP selon SEQ ID NO:1.

2. Procédé selon la revendication 1, **caractérisé en ce que** les deux anticorps se lient à des parties de séquence de la partie médiane de la NT-proANP qui s'étend de l'acide aminé 53 à l'acide aminé 83 de la NT-proANP.

3. Procédé selon les revendications 1 ou 2, **caractérisé en ce que** les anticorps sont des anticorps monoclonaux et/ou polyclonaux.

4. Procédé selon l'une des revendications 1 à 3, **caractérisé en ce que** les deux anticorps sont des anticorps polyclonaux purifiés par affinité.

5. Procédé selon l'une des revendications 1 à 4, **caractérisé en ce que** l'un des anticorps est obtenu par immunisation d'un animal avec un antigène qui contient une séquence synthétique de peptides qui comporte les acides aminés 53 à 72 de la SEQ ID NO:3 de la proANP, l'autre anticorps étant obtenu par immunisation par un antigène qui contient une séquence synthétique de peptides qui comporte les acides aminés 73 à 90 de la SEQ ID NO:4 de la proANP.

6. Procédé selon l'une des revendications 1 à 5, **caractérisé en ce que** l'un des anticorps est marqué et que l'autre anticorps est lié à une phase solide ou peut être lié sélectivement à une phase solide.

7. Procédé selon l'une des revendications 1 à 5, **caractérisé en ce que** tant le premier que le deuxième anticorps sont présents sous forme dispersée dans le mélange liquide de réaction et **en ce qu'**un premier composant de marquage lié au premier anticorps est une partie d'un système de marquage basé sur l'extinction ou l'amplification de la fluorescence de la chimiluminescence et **en ce que** le deuxième composant de marquage de ce système de marquage est lié au deuxième anticorps de telle sorte qu'un signal mesurable qui permet la détection des complexes sandwich formés dans la solution de mesure est créé après liaison des deux anticorps sur la proANP à détecter.

8. Procédé selon la revendication 7, **caractérisé en ce que** le système de marquage comporte des krypates ou chélates de terres rares combinés avec un colorant fluorescent ou chimioluminescent, en particulier de type cyanine.

9. Procédé selon l'une des revendications 1 à 8, **caractérisé en ce qu'**il est utilisé dans le domaine du diagnostic cardiaque.

10. Procédé selon la revendication 9, **caractérisé en ce qu'**il est réalisé dans le cadre d'une détermination de plusieurs paramètres dans laquelle plusieurs paramètres qui interviennent dans le diagnostic cardiaque, en particulier la BNP et la proBNP sont déterminés simultanément.

11. Procédé selon l'une des revendications 1 à 8, **caractérisé en ce qu'**il est utilisé pour le diagnostic, la détermination du degré de gravité et le pronostic ainsi que le contrôle de l'évolution de la thérapie d'une septicémie.

12. Procédé selon la revendication 11, **caractérisé en ce qu'**il est réalisé dans le cadre d'une détermination de plusieurs paramètres dans laquelle au moins un autre paramètre qui intervient dans le diagnostic de la septicémie est déterminé simultanément.

13. Procédé selon la revendication 12, **caractérisé en ce que** le ou les paramètres qui interviennent dans le diagnostic de la septicémie sont sélectionnés dans l'ensemble constitué des anticorps antigangliosides, des protéines procalcitonine, CA 125, CA 19-9, S100B, S100A, LASP-1, des fragments solubles de cytokératine, en particulier des CYFRA 21, de la TPS et/ou de fragments solubles de cytokératine-1 (sCY1F), des peptides inflammine et CHP, d'autres prohormones peptidiques, de la glycine-N-acyltransférase (GNAT), de la carbomoylphosphate synthétase 1 (CPS 1) et de la protéine C-réactive (CRP) ou de ses fragments.
